# EUROPÄISCHE PATENTSCHRIFT

(11) **EP 1 030 846 B1**
(45) Veröffentlichungstag und Bekanntmachung des Hinweises auf die Patenterteilung: **16.07.2003**
(21) Anmeldenummer: 98959829.7
(22) Anmeldetag: 03.11.1998
(51) Int. Cl.: C07D 239/54, C07D 239/46, C07C 275/24, C07C 275/16

(54) **VERFAHREN ZUR HERSTELLUNG VON AR(ALK)YLURACILEN, NEUE ZWISCHENPRODUKTE HIERFÜR UND VERFAHREN ZU DEREN HERSTELLUNG**
METHOD FOR PRODUCING AR(ALK)YLURACILES, NOVEL CORRESPONDING INTERMEDIATE PRODUCTS AND METHOD FOR PRODUCING SAID INTERMEDIATE PRODUCTS
PROCEDE DE PRODUCTION D'AR(ALK)YLURACILES, NOUVEAUX PRODUITS INTERMEDIAIRES CORRESPONDANTS ET PROCEDE DE PRODUCTION DE CES DERNIERS

(30) Priorität: 13.11.1997 DE 19750195
(43) Veröffentlichungstag der Anmeldung: 30.08.2000
(73) Patentinhaber: Bayer CropScience AG, 40789 Monheim (DE)
(72) Erfinder: GALLENKAMP, Bernd, D-42113 Wuppertal (DE); LANTZSCH, Reinhard, D-42115 Wuppertal (DE); ROHE, Lothar, D-42113 Wuppertal (DE); MULDER, Lubbertus, D-58135 Hagen (DE)
(86) Internationale Anmeldenummer: EP9806959
(87) Internationale Veröffentlichungsnummer: WO99025698

(56) Entgegenhaltungen:
- WO-A-95/32952
- DE-A- 19 604 582
- DE-A- 19 612 032
- US-A- 3 690 863
- EGLE M. BECALLI: "THE VILSMEYER-HAACK REACTION OF ISOXAZOLIN-5-ONES." JOURNAL OF ORGANIC CHEMISTRY., Bd. 52, Nr. 15, 1987, Seiten 3426-3434, XP002095148 EASTON US

## Beschreibung

Die Erfindung betrifft ein Verfahren zur Herstellung von Ar(alk)yluracilen, welche als herbizid wirksame Verbindungen bekannt sind, neue Zwischenprodukte hierfür und Verfahren zu deren Herstellung.

Es ist bekannt, daß bestimmte substituierte Cyanophenyluracile durch Umsetzung von entsprechenden Aminoalkensäureestern mit entsprechenden Cyanophenylisocyanaten oder mit Cyanophenylurethanen in Gegenwart von Reaktionshilfsmitteln, wie z.B. Natriumhydrid, hergestellt werden können (vgl. EP-A-648749). Bei dieser Verfahrensweise sind jedoch die Ausbeute und die Qualität der so erhaltenen Produkte nicht immer ganz zufriedenstellend und die benötigten Reaktionskomponenten für technische Belange wenig geeignet.

Weiter ist bekannt, daß bestimmte substituierte Phenyluracile durch Umsetzung von entsprechenden substituierten Aminoalkensäurephenylamiden mit geeigneten Kohlensäurederivaten hergestellt werden können (vgl. WO-A-95/32952). Der in dieser Publikation beschriebene Syntheseweg ist jedoch vielstufig und aufwendig.

Ferner ist bekannt, daß man substituierte Cyanophenyluracile erhält, wenn man entsprechende Cyanophenylpyrimidinone mit Basen umsetzt und die hierbei gebildeten substituierten Aminoalkensäure-cyanophenylamide mit Kohlensäurederivaten, wie z.B. Phosgen oder Diphenylcarbonat umsetzt (vgl. DE-A-19604582). Hierbei werden die gewünschten Produkte jedoch nicht immer in hohen Ausbeuten und in befriedigender Qualität erhalten.

### Gegenstand der Erfindung sind

(a) ein Verfahren zur Herstellung von Ar(alk)yluracilen der allgemeinen Formel (I) 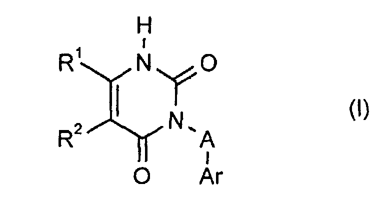 in welcher
   - A: für eine Einfachbindung oder für geradkettiges oder verzweigtes Alkandiyl mit 1 bis 4 Kohlenstoffatomen,
   - Ar: für jeweils gegebenenfalls durch Amino, Cyano, Halogen oder die Gruppierung -N(R³)SO₂R⁴ substituiertes Phenyl oder Naphthyl,
   - R¹: für gegebenenfalls durch Fluor und/oder Chlor substituiertes Alkyl mit1 bis 4 Kohlenstoffatomen,
   - R²: für Wasserstoff, Fluor, Chlor, Brom oder Alkyl mit 1 bis 4 Kohlenstoffatomen,
   - R³: für Wasserstoff oder für jeweils gegebenenfalls durch Fluor und/oder Chlor substituiertes Alkyl, Alkylcarbonyl oder Alkylsulfonyl mit jeweils 1 bis 6 Kohlenstoffatomen in den Alkylgruppen, für jeweils gegebenenfalls durch Fluor und/oder Chlor substituiertes Cycloalkylcarbonyl oder Cycloalkylsulfonyl mit jeweils 3 bis 6 Kohlenstoffatomen in den Cycloalkylgruppen, oder für jeweils gegebenenfalls durch Fluor und/oder Chlor substituiertes Phenylcarbonyl oder Phenylsulfonyl, und
   - R⁴: für gegebenenfalls durch Fluor und/oder Chlor substituiertes Alkyl mit 1 bis 6 Kohlenstoffatomen, für gegebenenfalls durch Fluor und/oder Chlor substituiertes Cycloalkyl mit 3 bis 6 Kohlenstoffatomen, oder für jeweils gegebenenfalls durch Fluor und/oder Chlor substituiertes Phenyl steht,
   dadurch gekennzeichnet, dass man substituierte Aminoalkensäureamide der allgemeinen Formeln (IIa) oder (IIb) 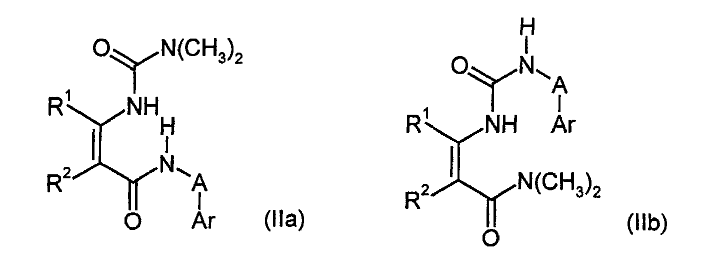 in welchen
   A, Ar, R¹ und R² die oben angegebene Bedeutung haben,
   gegebenenfalls in Gegenwart eines Verdünnungsmittels auf Temperaturen zwischen 40°C und 120°C erwärmt,
(b) neue substituierte Aminoalkensäureamide der allgemeinen Formeln (IIa) oder (IIb) 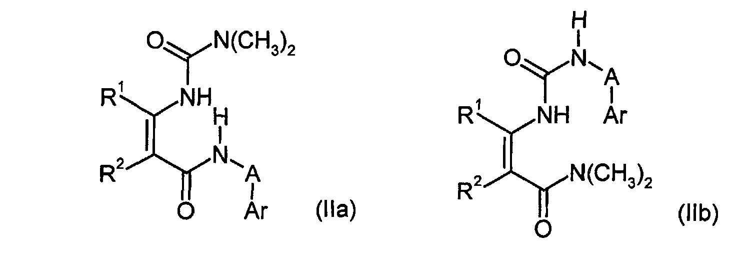 in welchen
   A, Ar, R¹ und R² die oben angegebene Bedeutung haben,
(c) ein Verfahren zur Herstellung von substituierten Aminoalkensäureamiden der allgemeinen Formel (IIa), dadurch gekennzeichnet, daß man substituierte Pyrimidone der allgemeinen Formel (III) 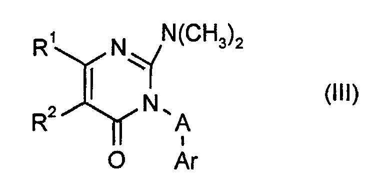 in welcher
   A, Ar, R¹ und R² die oben angegebene Bedeutung haben,
   mit einer Säure gegebenenfalls in Gegenwart eines Verdünnungsmittels bei Temperaturen zwischen 0°C und 100°C umsetzt,
(d) ein Verfahren zur Herstellung von substituierten Aminoalkensäureamiden der allgemeinen Formel (IIb), dadurch gekennzeichnet, daß man substituierte Oxazinone der allgemeinen Formel (IV) 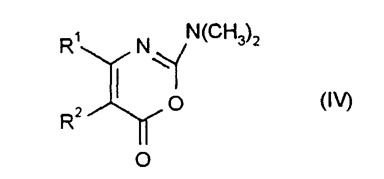 in welcher
   R¹ und R² die oben angegebene Bedeutung haben
   mit Ar(alk)ylaminen der allgemeinen Formel (V)

   H₂N-A-Ar (V)

   in welcher
   A und Ar die oben angegebene Bedeutung haben,
   gegebenenfalls in Gegenwart eines Verdünnungsmittels bei Temperaturen zwischen 0°C und 150°C umsetzt,
(e) neue substituierte Pyrimidone der allgemeinen Formel (III) 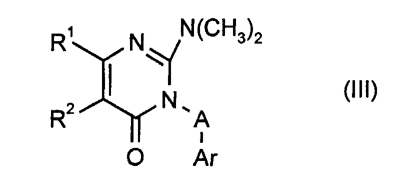 in welcher
   A, Ar, R¹ und R² die oben angegebene Bedeutung haben,
(f) ein Verfahren zur Herstellung von substituierten Pyrimidonen der allgemeinen Formel (III), dadurch gekennzeichnet, dass man substituierte Aminoalkensäureamide der allgemeinen Formel (VI) 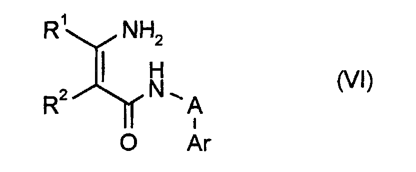 in welcher
   A, Ar, R¹ und R² die oben angegebene Bedeutung haben,
   mit Dichlormethylen-dimethylimmoniumchlorid der Formel (VII)

   Cl₂C=N(CH₃)₂ Cl (VII)

   in Gegenwart eines Verdünnungsmittels bei Temperaturen zwischen 0°C und 100°C umsetzt.

Gegenstand der Erfindung ist auch der neue Weg zur Herstellung von Ar(alk)yluracilen der allgemeinen Formel (I), bei dem die oben unter (f), (c) und (a) allgemein dargelegten Stufen durchlaufen werden.

Es ist als überraschend zu bezeichnen, dass durch diesen neuen Weg die Ar(alk)yluracile der allgemeinen Formel (I) auf einfache Weise in hohen Ausbeuten und in guter Qualität hergestellt werden können.

Die gegebenenfalls als Vorprodukte zu verwendenden Oxazinone der allgemeinen Formel (IV) sind bekannt und/oder können nach an sich bekannten Verfahren hergestellt werden (vgl. Bull. Soc. Chim. Belg. 101 (1992), 313-321; Herstellungsbeispiele).

Die gegebenenfalls als Vorprodukte zu verwendenden substituierten Aminoalkensäureamide der allgemeinen Formel (VI) sind bekannt und/oder können nach an sich bekannten Verfahren hergestellt werden (vgl. DE-A-19604582; Herstellungsbeispiele).

In den Formeln (I), (IIa), (IIb), (III) und (IV) stehen vorzugsweise
- A: für eine Einfachbindung oder für eine Methylengruppe,
- Ar: für gegebenenfalls durch Amino, Cyano, Fluor, Chlor, Brom oder die Gruppierung -N(R³)SO₂R⁴ substituiertes Phenyl,
- R¹: für jeweils gegebenenfalls durch Fluor und/oder Chlor substituiertes Methyl oder Ethyl,
- R²: für Wasserstoff, Fluor, Chlor, Brom, Methyl oder Ethyl,
- R³: für Wasserstoff oder für jeweils gegebenenfalls durch Fluor und/oder Chlor substituiertes Methyl, Ethyl, n- oder i-Propyl, Acetyl, Propionyl, Methylsulfonyl oder Ethylsulfonyl, für jeweils gegebenenfalls durch Fluor und/oder Chlor substituiertes Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl, oder für jeweils gegebenenfalls durch Fluor und/oder Chlor substituiertes Phenylcarbonyl oder Phenylsulfonyl, und
- R⁴: für jeweils gegebenenfalls durch Fluor und/oder. Chlor substituiertes Methyl, Ethyl, n- oder i-Propyl, n-, i-, s- oder t-Butyl, für jeweils gegebenenfalls durch Fluor und/oder Chlor substituiertes Cyclopropyl, Cyclobutyl, Cyclopentyl oder Cyclohexyl, oder für jeweils gegebenenfalls durch Fluor und/oder Chlor substituiertes Phenyl.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Die oben unter (a), (c), (d) und (f) beschriebenen erfindungsgemäßen Verfahren werden im folgenden abkürzend als "Verfahren (a)", "Verfahren (c)", "Verfahren (d)" und "Verfahren (e)" bezeichnet.

Verwendet man beispielsweise N-(4-Cyano-2,5-difluor-phenyl)-3-amino-4,4,4-trifluor-2-butensäureamid und Dichlormethylen-dimethylimmoniumchlorid als Ausgangsstoffe, setzt das bei deren Umsetzung gemäß Verfahren (f) erhaltene Produkt beispielsweise mit Hydrogenbromid gemäß Verfahren (c) um und erwärmt das hierbei erhaltene Produkt gemäß Verfahren (a), so kann der Reaktionsablauf durch das folgende Formelschema skizziert werden:

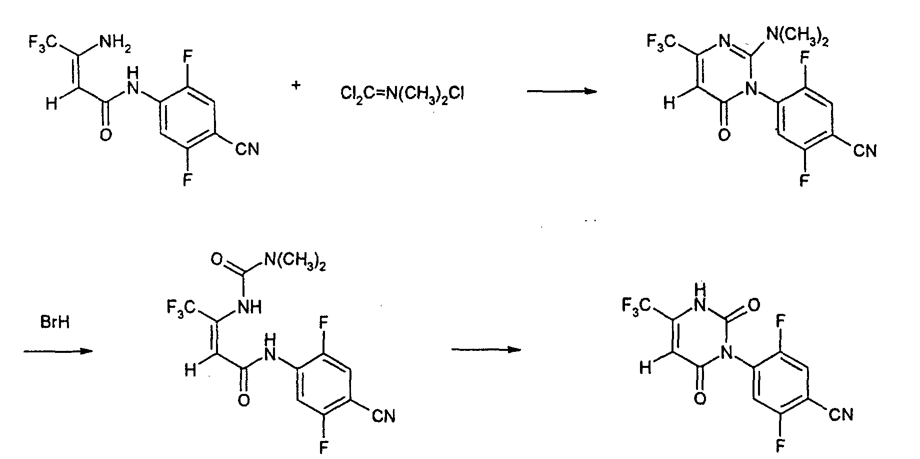

Das erfindungsgemäße Verfahren (f) zur Herstellung von Verbindungen der allgemeinen Formel (III) wird unter Verwendung eines Verdünnungsmittels durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Carbonsäuren, wie Essigsäure oder Propionsäure, Nitrile, wie Acetonitril, Propionitril oder Butyronitril; Ester wie Essigsäuremethylester oder Essigsäureethylester, Amide, wie N,N-Dimethylformamid, N,N-Dimethylacetamid, N-Methyl-formanilid, N-Methyl-pyrrolidon oder Hexamethylphosphorsäuretriamid, Sulfoxide, wie Dimethylsulfoxid.

Die Reaktionstemperaturen können bei der Durchführung des erfindungsgemäßen Verfahrens (f) in einem größeren Bereich variiert werden. Im allgemeinen arbeitet man bei Temperaturen zwischen 0°C und 100°C, vorzugsweise zwischen 20°C und 80°C.

Das erfindungsgemäße Verfahren (f) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens (f) setzt man pro Mol an Ausgangsverbindung der Formel (VI) im allgemeinen 1,0 bis 3,0 Mol, vorzugsweise 1,1 bis 2,0 Mol an Dichlormethylen-dimethylimmoniumchlorid der Formel (VII) ein.

In einer bevorzugten Ausführungsform des erfindungsgemäßen Verfahrens (f) wird das Dichlormethylen-dimethylimmoniumchlorid der Formel (VII) in einem Verdünnungsmittel vorgelegt, und ein substiutiertes Aminoalkensäure-cyanophenylamid der allgemeinen Formel (VI) wird bei Raumtemperatur (etwa 20°C bis 30°C) unter Rühren portionsweise dazu gegeben. Die Reaktionsmischung wird dann mehrere Stunden bei etwas erhöhter Temperatur gerührt, bis die Gasentwicklung abgeklungen ist. Die Aufarbeitung kann nach üblichen Methoden erfolgen. Beispielsweise wird mit wässriger Natriumhydrogencarbonat-Lösung verrührt, die organische Phase abgetrennt, mit wässriger Natriumhydrogencarbonat-Lösung und dann mit Wasser gewaschen und unter vermindertem Druck eingeengt. Das im Rückstand verbleibende Produkt kann durch Digerieren mit einem organischen Lösungsmittel zur Kristallisation gebracht werden (vgl. die Herstellungsbeispiele).

Das erfindungsgemäße Verfahren (c) zur Herstellung von Verbindungen der allgemeinen Formel (IIa) wird unter Verwendung einer Säure durchgeführt. Unter Säuren sind in diesem Zusammenhang vorzugsweise Protonensäuren zu verstehen. Hierzu gehören beispielsweise Hydrogenfluorid, Hydrogenchlorid, Hydrogenbromid, Hydrogeniodid, Schwefelsäure, Phosphorsäure, Methansulfonsäure, Trifluormethansulfonsäure, Benzolsulfonsäure, p-Toluolsulfonsäure, Ameisensäure, Essigsäure, Propionsäure und Trifluoressigsäure. Hydrogenchlorid wird als Säure beim erfindungsgemäße Verfahren (c) ganz besonders bevorzugt.

Die erfindungsgemäßen Verfahren (c) und (d) werden vorzugsweise unter Verwendung von Verdünnungsmitteln durchgeführt. Als Verdünnungsmittel kommen hierbei neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethylether oder -diethylether; Ketone, wie Aceton, Butanon oder Methyl-isobutyl-keton; Carbonsäuren, wie Ameisensäure, Essigsäure oder Propionsäure, Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Als besonders bevorzugtes Verdünnungsmittel für Verfahren (c) sei N,N-Dimethylformamid, als besonders bevorzugtes Verdünnungsmittel für die Verfahren (d) Ethylenglykol-dimethylether (1,2-Dimethoxy-ethan) genannt.

Die Reaktionstemperaturen können bei der Durchführung der erfindungsgemäßen Verfahren (c) und (d) jeweils in einem größeren Bereich variiert werden. Im allgemeinen arbeitet man bei Temperaturen zwischen 0°C und 150°C, vorzugsweise zwischen 10°C und 110°C.

Die erfindungsgemäßen Verfahren (c) und (d) werden im allgemeinen unter Normaldruck durchgeführt. Es ist jedoch auch möglich, die erfindungsgemäßen Verfahren (c) und (d) unter erhöhtem oder vermindertem Druck - im allgemeinen zwischen 0,1 bar und 10 bar - durchzuführen.

Zur Durchführung des erfindungsgemäßen Verfahrens (c) setzt man pro Mol an Cyanophenylpyrimidon der Formel (III) im allgemeinen 0,8 bis 1,5 Mol, vorzugsweise 0,9 bis 1,2 Mol einer Säure ein.

In einer bevorzugten Ausführungsform des erfindungsgemäßen Verfahrens (c) wird ein Cyanophenylpyrimidon der Formel (III) in einem Lösungsmittel vorgelegt und eine Säure wird bei Raumtemperatur (etwa 20°C bis 30°C) langsam eindosiert. Die Reaktionsmischung wird dann noch einige Stunden gerührt und anschließend auf übliche Weise aufgearbeitet. Beispielsweise wird die Mischung auf Eiswasser gegossen und gut durchgerührt. Das hierbei im allgemeinen kristallin anfallende Produkt kann durch Absaugen isoliert werden (vgl. die Herstellungsbeispiele).

Zur Durchführung des erfindungsgemäßen Verfahrens (d) setzt man pro Mol an Oxazinon der Formel (IV) im allgemeinen 0,8 bis 1,5 Mol, vorzugsweise 0,9 bis 1,2 Mol eines Ar(alk)ylamins der Formel (V) ein.

In einer bevorzugten Ausführungsform des erfindungsgemäßen Verfahrens (d) wird ein Oxazinon der Formel (IV) mit einem Ar(alk)ylamin der Formel (V) in einem Lösungsmittel bei Raumtemperatur vermischt, die Reaktionsmischung wird bis zum Ende der Umsetzung bei erhöhter Temperatur gerührt. Das Umsetzungsprodukt fällt im allgemeinen bei Erkalten kristallin an und kann durch Absaugen isoliert werden (vgl. die Herstellungsbeispiele).

Das erfindungsgemäße Verfahren (a) zur Herstellung von Verbindungen der allgemeinen Formel (I) wird vorzugsweise unter Verwendung eines Verdünnungsmittels durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung des erfindungsgemäßen Verfahrens (a) in einem größeren Bereich variiert werden. Im allgemeinen arbeitet man bei Temperaturen zwischen 40°C und 120°C, vorzugsweise zwischen 50°C und 100°C.

Das erfindungsgemäße Verfahren (a) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

In einer bevorzugten Ausführungsform des erfindungsgemäßen Verfahrens (a) wird ein substituiertes Aminoalkensäureamid der allgemeinen Formeln (IIa) oder (IIb) mit einem Verdünnungsmittel bei Raumtemperatur (etwa 20°C bis 30°C) verrührt und die Mischung wird unter Rühren erwärmt, bis die Umsetzung abgeschlossen ist. Die Aufarbeitung wird dann auf übliche Weise durchgeführt (vgl. die Herstellungsbeispiele).

Die erfindungsgemäß herzustellenden substituierten Ar(alk)yluracile der allgemeinen Formel (I) sind bereits als herbizid wirksame Verbindungen bekannt (vgl. EP-A-648 749).

### Herstellungsbeispiele:

### Beispiel (I-1)

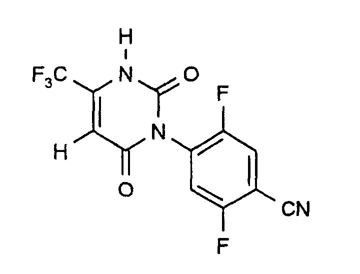

### (Verfahren (a))

Eine Mischung aus 71,0 g (0,17 Mol) N-(4-Cyano-2,5-difluor-phenyl)-3-(dimethylaminocarbonylamino)-4,4,4-trifluor-2-butensäureamid und 350 ml 1,2-Dimethoxy-ethan (Ethylenglycol-dimethylether) wird unter Rühren langsam auf 70°C erwärmt und drei Stunden bei dieser Temperatur gerührt. Anschließend wird im Wasserstrahlvakuum eingeengt und nach dem Verrühren des Rückstands mit 85 g Methylenchlorid erneut eingeengt. Der Rückstand wird mit 765 ml Wasser verrührt und 30 Minuten stehen gelassen. Dann wird das Wasser weitgehend abdekantiert, das zurückbleibende Rohprodukt mit 380 ml Wasser intensiv ("Uitra-Turrax") verrührt und filtriert. Die vereinigten wässrigen Lösungen werden unter Rühren mit 15 ml konz. Salzsäure tropfenweise versetzt und das hierbei kristallin angefallene Produkt wird durch Absaugen isoliert und unter vermindertem Druck bei 50°C getrocknet.

Man erhält 49,8 g (92,4%ig, d.h. 87% der Theorie) 1-(4-Cyano-2,5-difluor-phenyl)-3,6-dihydro-2,6-dioxo-4-trifluormethyl-1(2H)-pyrimidin vom Schmelzpunkt 208°C.

### Beispiel (I-2)

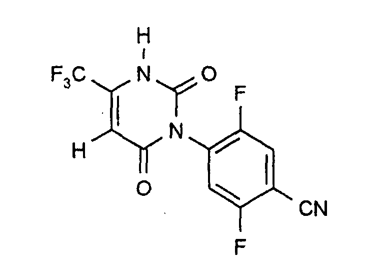

### (Verfahren (a))

Eine Mischung aus 1,8 g (5 mMol) N,N-Dimethyl-3-(4-cyano-2,5-difluor-phenylamino-carbonylamino)-4,4,4-trifluor-2-butensäureamid und 20 ml Essigsäure wird 16 Stunden unter Rückfluß erhitzt und nach Abkühlen mit Wasser langsam auf etwa das dreifache Volumen verdünnt. Das hierbei kristallin angefallene Produkt wird durch Absaugen isoliert.

Man erhält 1,25 g (79% der Theorie) 1-(4-Cyano-2,5-difluor-phenyl)-3,6-dihydro-2,6-dioxo-4-trifluormethyl-1(2H)-pyrimidin vom Schmelzpunkt 217°C.

### Beispiel (IIa-1)

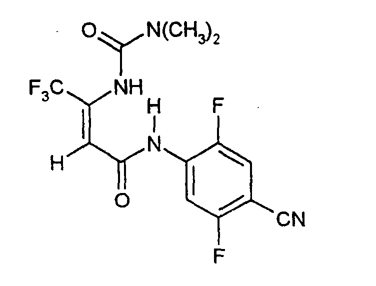

### (Verfahren (c))

Eine Mischung aus 73,3 g (0,20 Mol) 3-(4-Cyano-2,5-difluor-phenyl)-2-dimethylamino-6-trifluormethyl-pyrimidin-4-on und 300 ml N,N-Dimethyl-formamid wird bei Raumtemperatur (etwa 20°C bis 30°C) unter Rühren mit 9 ml konz. Salzsäure tropfenweise versetzt, im angegebenen Temperaturbereich noch ca. drei Stunden gerührt und dann in 1800 ml Eiswasser eingerührt. Das hierbei kristallin angefallene Produkt wird durch Absaugen isoliert.

Man erhält 71,1 g (84,5%ig, d.h. 83% der Theorie) N-(4-Cyano-2,5-difluor-phenyl)-3-(dimethylaminocarbonylamino)-4,4,4-trifluor-2-butensäureamid vom Schmelzpunkt 138°C.

### Beispiel (IIb-1)

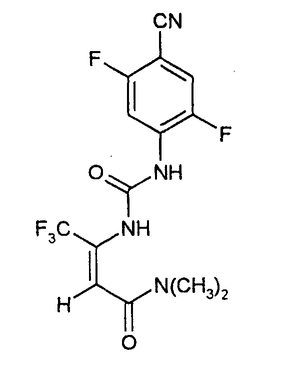

### (Verfahren (d))

Eine Lösung von 4,2 g (20 mMol) 2-Dimethylamino-4-trifluormethyl-1,3-oxazin-6-on und 3,1 g (20 mMol) 4-Cyano-2,5-difluor-anilin in 40 ml 1,2-Dimethoxy-ethan wird 24 Stunden unter Rühren zum Sieden erhitzt. Nach Abkühlen wird das kristallin angefallene Produkt durch Absaugen isoliert.

Man erhält 3,8 g (52,5% der Theorie) N,N-Dimethyl-3-(4-cyano-2,5-difluor-phenylamino-carbonylamino)-4,4,4-trifluor-2-butensäureamid vom Schmelzpunkt 198°C.

### Beispiel (IIb-2)

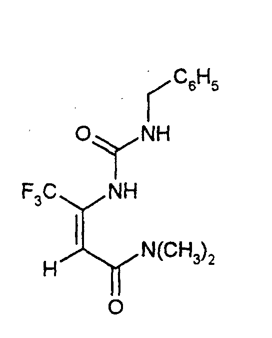

### (Verfahren (d))

Eine Lösung von 1,0 g (5 mMol) 2-Dimethylamino-4-trifluormethyl-1,3-oxazin-6-on und 0,54 g (5 mMol) Benzylamin in 10 ml 1,2-Dimethoxy-ethan wird 2 Stunden bei 25°C gerührt. Anschließend wird im Wasserstrahlvakuum eingeengt, der Rückstand in Methylenchlorid aufgenommen und über Kieselgel filtriert. Vom Filtrat wird das Lösungsmittel im Wasserstrahlvakuum sorgfältig abdestilliert.

Man erhält 1,0 g (95,8%ig, d.h. 67% der Theorie) N,N-Dimethyl-3-(benzylaminocarbonylamino)-4,4,4-trifluor-2-butensäureamid als öligen Rückstand.

### Beispiel (III-1)

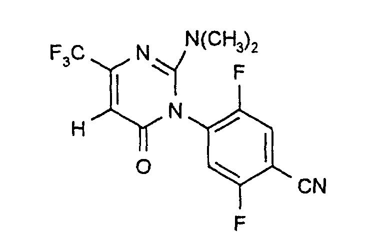

156 g (0,96 Mol) Dichlormethylen-dimethylimmoniumchlorid (Phosgenimmoniumchlorid) werden in 1200 ml Chloroform vorgelegt und bei Raumtemperatur (etwa 20°C bis 30°C) unter Rühren mit 203 g (0,60 Mol) N-(4-Cyano-2,5-difluor-phenyl)-3-amino-4,4,4-trifluor-2-butensäureamid portionsweise versetzt. Dann wird die Reaktionsmischung 90 Minuten bei 40°C (Gasentwicklung) und zwei Stunden unter Rückfluß gerührt. Anschließend wird bei Raumtemperatur mit 170 ml gesättigter wässriger Natriumhydrogencarbonat-Lösung verrührt, die organische Phase abgetrennt mit gesättigter wässriger Natriumhydrogencarbonat-Lösung und dann mit Wasser gewaschen und im Wasserstrahlvakuum eingeengt. Der Rückstand wird mit 300 ml Diisopropylether verrührt und das kristalline Produkt durch Absaugen isoliert.

Man erhält 189 g (93,9%ig, d.h. 86% der Theorie) 3-(4-Cyano-2,5-difluor-phenyl)-2-dimethylamino-6-trifluormethyl-pyrimidin-4-on vom Schmelzpunkt 147°C.

### Ausgangsstoffe der Formel (IV):

### Beispiel (IV-1)

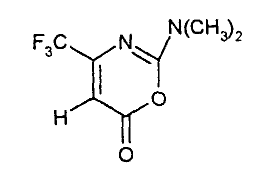

24,4 g (150 mMol) Dichlormethylen-dimethylimmoniumchlorid werden in 130 ml 1,2-Dimethoxy-ethan suspendiert und bei 25°C innerhalb von ca. 40 Minuten mit einer Lösung von 25 g (136 mMol) 4,4,4-Trifluor-2-butensäure-ethylester in 130 ml 1,2-Dimethoxy-ethan unter Rühren tropfenweise versetzt. Die Reaktionsmischung wird 12 Stunden unter Rühren zum Sieden erhitzt. Nach Abkühlen wird das Lösungsmittel im Wasserstrahlvakuum sorgfältig abdestilliert, der kristalline Rückstand (27 g) mit Hexan digeriert und das Produkt durch Absaugen isoliert.

Man erhält 22,6 g (80% der Theorie) 2-Dimethylamino-4-trifluormethyl-1,3-oxazin-6-on vom Schmelzpunkt 79°C.

### Ausgangsstoffe der Formel (VI):

### Beispiel (VI-1)

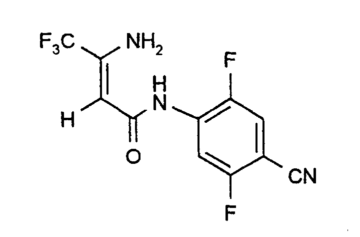

### Stufe 1

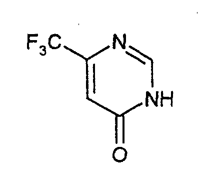

Eine Mischung aus 564 g (3,0 Mol) 4,4,4-Trifluor-acetessigsäure-ethylester, 472 g (4,5 Mol) Formamidin-acetat und 3800 ml Methanol wird bei Raumtemperatur (ca. 20°C) unter Rühren mit 382 g (3,81 Mol) Natriumcarbonat portionsweise versetzt und die Reaktionsmischung wird 15 Stunden bei 60°C gerührt. Anschließend wird bei 5°C bis 10°C mit 780 ml konz. Salzsäure angesäuert und dann im Wasserstrahlvakuum eingeengt. Der Rückstand wird mit 2700 ml Essigsäureethylester und 1200 ml Wasser verrührt, die organische Phase abgetrennt und hiervon das Lösungsmittel im Wasserstrahlvakuum sorgfältig abdestilliert.

Man erhält 473 g (100%ig, d.h. 96% der Theorie) 6-Trifluormethyl-pyrimidin-4-on als festen Rückstand vom Schmelzpunkt 169°C.

### Stufe 2

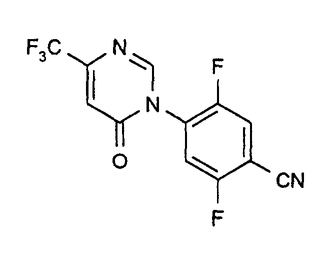

### Variante (a)

Eine Lösung von 472 g (2,88 Mol) 6-Trifluormethyl-pyrimidin-4-on in 2300 ml Dimethylsulfoxid wird bei Raumtemperatur (etwa 20°C bis 30°C) unter Rühren mit 398 g (2,88 Mol) Kaliumcarbonat portionsweise versetzt und die Mischung wird 20 Minuten bei Raumtemperatur gerührt. Dann werden 466 g (2,88 Mol) 2,4,5-Trifluorbenzonitril portionsweise dazu gegeben, die Reaktionsmischung wird 18 Stunden bei 60°C gerührt und anschließend zu 9 Liter Eiswasser gegeben. Das kristallin angefallene (Roh-)Produkt wird nach etwa einer Stunde durch Absaugen isoliert und im Wasserstrahlvakuum getrocknet. Zur Reinigung wird 5 Stunden mit 3 Liter Isopropanol/Wasser (1:1) gerührt, erneut abgesaugt und im Wasserstrahlvakuum bei 40°C getrocknet.

Man erhält 655 g (80,2%ig, d.h. 63% der Theorie) 3-(4-Cyano-2,5-difluor-phenyl)-6-trifluormethyl-pyrimidin-4-on vom Schmelzpunkt 144°C.

### Variante (b)

Eine Lösung von 32,8 g (0,20 Mol) 6-Trifluormethyl-pyrimidin-4-on in 160 ml Dimethylsulfoxid wird bei Raumtemperatur (etwa 20°C bis 30°C) unter Rühren mit 22,4 g (0,20 Mol) Kalium-t-butylat portionsweise versetzt und die Mischung wird 20 Minuten bei Raumtemperatur gerührt. Dann werden 35,5 g (0,25 Mol) 2,4,5-Trifluorbenzonitril portionsweise dazu gegeben, die Reaktionsmischung wird 10 Stunden bei 60°C bis 70°C gerührt und anschließend zu 650 ml Eiswasser gegeben. Das kristallin angefallene (Roh-)Produkt wird nach etwa einer Stunde durch Absaugen isoliert und im Wasserstrahlvakuum getrocknet. Zur Reinigung wird 10 Minuten mit 600 ml Isopropanol und 300 ml Wasser gerührt, erneut abgesaugt und im Wasserstrahlvakuum bei 40°C getrocknet.

Man erhält 42,9 g (89,9%ig, d.h. 64% der Theorie) 3-(4-Cyano-2,5-difluor-phenyl)-6-trifluormethyl-pyrimidin-4-on vom Schmelzpunkt 144°C.

### Stufe 3

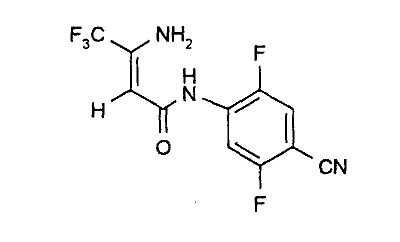

652 g (1,75 Mol) 3-(4-Cyano-2,5-difluor-phenyl)-6-trifluormethyl-pyrimidin-4-on werden in 9 Liter Wasser suspendiert und bei Raumtemperatur (etwa 20°C bis 30°C) mit 201 ml konz. Natronlauge (3,33 Mol NaOH) tropfenweise versetzt. Die Reaktionsmischung wird 15 Stunden bei Raumtemperatur gerührt und anschließend abgesaugt. Der Filterkuchen wird in 2300 ml Toluol aufgenommen und die Lösung am Wasserabscheider getrocknet. Das beim Abkühlen (zuletzt auf ca. 5°C) kristallin anfallende Produkt wird durch Absaugen isoliert.

Man erhält 530 g (86,1%ig, d.h. 89,5% der Theorie) N-(4-Cyano-2,5-difluor-phenyl)-3-amino-4,4,4-trifluor-2-butensäureamid vom Schmelzpunkt 170°C.

## Patentansprüche

1. Verfahren zur Herstellung von Ar(alk)yluracilen der allgemeinen Formel (I) 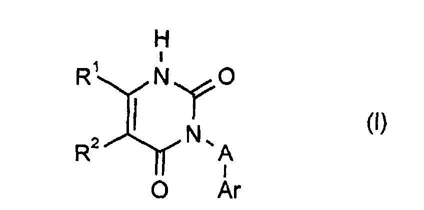 in welcher
A für eine Einfachbindung oder für geradkettiges oder verzweigtes Alkandiyl mit 1 bis 4 Kohlenstoffatomen steht,
Ar für jeweils gegebenenfalls durch Amino, Cyano, Halogen oder die Gruppierung -N(R³)SO₂R⁴ substituiertes Phenyl oder Naphthyl steht,
R¹ für gegebenenfalls durch Fluor und/oder Chlor substituiertes Alkyl mitl bis 4 Kohlenstoffatomen steht,
R² für Wasserstoff, Fluor, Chlor, Brom oder Alkyl mit 1 bis 4 Kohlenstoffatomen steht,
R³ für Wasserstoff oder für jeweils gegebenenfalls durch Fluor und/oder Chlor substituiertes Alkyl, Alkylcarbonyl oder Alkylsulfonyl mit jeweils 1 bis 6 Kohlenstoffatomen in den Alkylgruppen, für jeweils gegebenenfalls durch Fluor und/oder Chlor substituiertes Cycloalkylcarbonyl oder Cycloalkylsulfonyl mit jeweils 3 bis 6 Kohlenstoffatomen in den Cycloalkylgruppen, oder für jeweils gegebenenfalls durch Fluor und/oder Chlor substituiertes Phenylcarbonyl oder Phenylsulfonyl steht, und
R⁴ für gegebenenfalls durch Fluor und/oder Chlor substituiertes Alkyl mit 1 bis 6 Kohlenstoffatomen, für gegebenenfalls durch Fluor und/oder Chlor substituiertes Cycloalkyl mit 3 bis 6 Kohlenstoffatomen, oder für jeweils gegebenenfalls durch Fluor und/oder Chlor substituiertes Phenyl steht,
**dadurch gekennzeichnet, dass** man substituierte Aminoalkensäureamide der allgemeinen Formeln (IIa) oder (IIb) 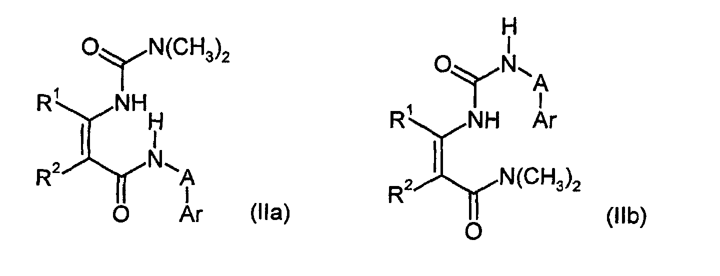 in welchen
A, Ar, R¹ und R² die oben angegebene Bedeutung haben,
gegebenenfalls in Gegenwart eines Verdünnungsmittels auf Temperaturen zwischen 40°C und 120°C erwärmt.

2. Verfahren nach Anspruch 1, **dadurch gekennzeichnet, dass**
A für eine Einfachbindung oder für eine Methylengruppe steht,
Ar für gegebenenfalls durch Amino, Cyano, Fluor, Chlor, Brom oder die Gruppierung -N(R³)SO₂R⁴ substituiertes Phenyl steht,
R¹ für jeweils gegebenenfalls durch Fluor und/oder Chlor substituiertes Methyl oder Ethyl steht,
R² für Wasserstoff, Fluor, Chlor, Brom, Methyl oder Ethyl steht,
R³ für Wasserstoff oder für jeweils gegebenenfalls durch Fluor und/oder Chlor substituiertes Methyl, Ethyl, n- oder i-Propyl, Acetyl, Propionyl, Methylsulfonyl oder Ethylsulfonyl, für jeweils gegebenenfalls durch Fluor und/oder Chlor substituiertes Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl, oder für jeweils gegebenenfalls durch Fluor und/oder Chlor substituiertes Phenylcarbonyl oder Phenylsulfonyl steht, und
R⁴ für jeweils gegebenenfalls durch Fluor und/oder Chlor substituiertes Methyl, Ethyl, n- oder i-Propyl, n-, i-, s- oder t-Butyl, für jeweils gegebenenfalls durch Fluor und/oder Chlor substituiertes Cyclopropyl, Cyclobutyl, Cyclopentyl oder Cyclohexyl, oder für jeweils gegebenenfalls durch Fluor und/oder Chlor substituiertes Phenyl steht.

3. Substituierte Aminoalkensäureamide der allgemeinen Formeln (IIa) oder (IIb) 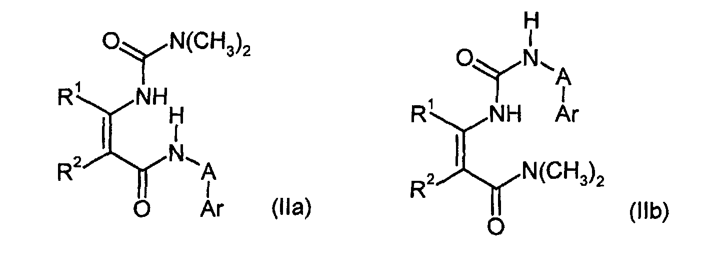 in welchen
A, Ar, R¹ und R² die Anspruch 1 oder 2 angegebene Bedeutung haben.

4. Verfahren zur Herstellung von substituierten Aminoalkensäureamiden der allgemeinen Formel (IIa), 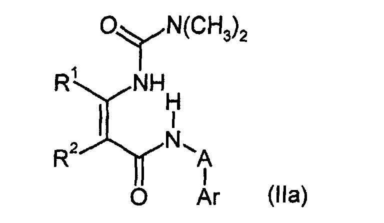 **dadurch gekennzeichnet, dass** man substituierte Pyrimidone der allgemeinen Formel (III) 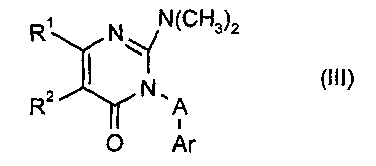 in welcher
A, Ar, R¹ und R² die in Anspruch 1 oder 2 angegebene Bedeutung haben,
mit einer Säure gegebenenfalls in Gegenwart eines Verdünnungsmittels bei Temperaturen zwischen 0°C und 100°C umsetzt.

5. Verfahren zur Herstellung von substituierten Aminoalkensäureamiden der allgemeinen Formel (IIb), 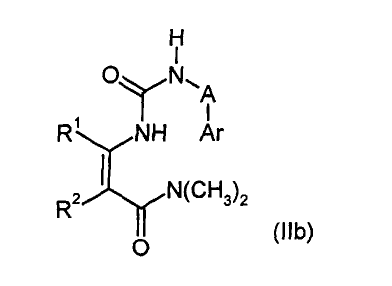 **dadurch gekennzeichnet, dass** man substituierte Oxazinone der allgemeinen Formel (IV) 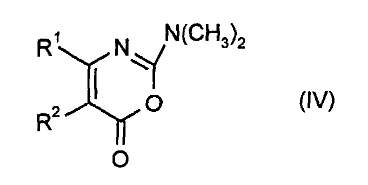 in welcher
R¹ und R² die in Anspruch 1 oder 2 angegebene Bedeutung haben
mit Ar(alk)ylaminen der allgemeinen Formel (V)
H₂N-A-Ar (V)
in welcher
A und Ar die in Anspruch 1 oder 2 angegebene Bedeutung haben,
gegebenenfalls in Gegenwart eines Verdünnungsmittels bei Temperaturen zwischen 0°C und 150°C umsetzt.

6. Substituierte Pyrimidone der allgemeinen Formel (III) 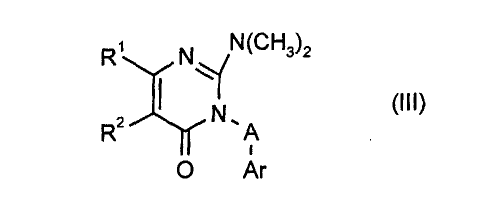 in welcher
A, Ar, R¹ und R² die in Anspruch 1 oder 2 angegebene Bedeutung haben.

7. Verfahren zur Herstellung von substituierten Pyrimidonen der allgemeinen Formel (III), 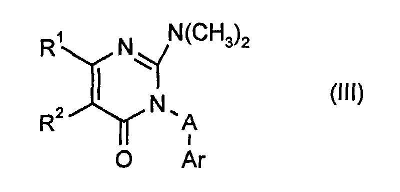 **dadurch gekennzeichnet, dass** man substituierte Aminoalkensäureamide der allgemeinen Formel (VI) 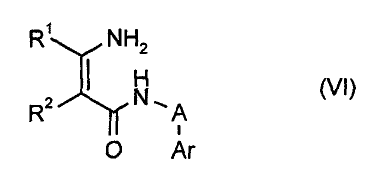 in welcher
A, Ar, R¹ und R² die in Anspruch 1 oder 2 angegebene Bedeutung haben,
mit Dichlormethylen-dimethylimmoniumchlorid der Formel (VII)
Cl₂C=N(CH₃)₂ Cl (VII)
in Gegenwart eines Verdünnungsmittels bei Temperaturen zwischen 0°C und 100°C umsetzt.

8. Verfahren zur Herstellung von Ar(alk)yluracilen der allgemeinen Formel (I) 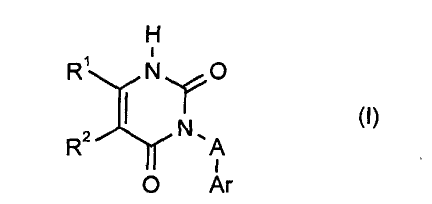 in welcher
A, Ar, R¹ und R² die in Anspruch 1 oder 2 angegebene Bedeutung haben,
**gekennzeichnet durch** die folgenden Schritte:
A) Umsetzung von substituierten Aminoalkensäureamiden der allgemeinen Formel (VI) 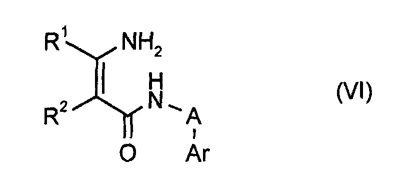 in welcher
A, Ar, R¹ und R² die in Anspruch 1 oder 2 angegebene Bedeutung haben,
mit Dichlormethylen-dimethylimmoniumchlorid der Formel (VII)
Cl₂C=N(CH₃)₂ Cl (VII)
in Gegenwart eines Verdünnungsmittels bei Temperaturen zwischen 0°C und 100°C;
B) Umsetzen der im Schritt A) hergestellten substituierten Pyrimidone der allgemeinen Formel (III) 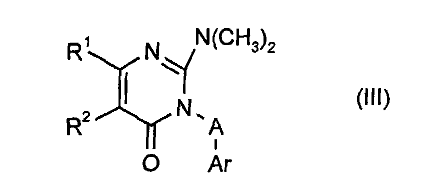 in welcher
A, Ar, R¹ und R² die in Anspruch 1 oder 2 angegebene Bedeutung haben,
mit einer Säure gegebenenfalls in Gegenwart eines Verdünnungsmittels bei Temperaturen zwischen 0°C und 100°C und
C) Erwärmen der im Schritt B) hergestellten substituierten Aminoalkensäureamide der allgemeinen Formeln (IIa) 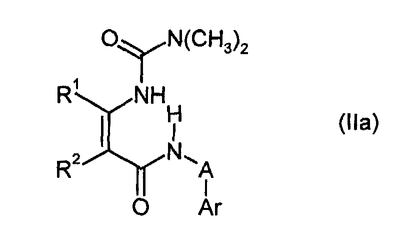
in welchen
A, Ar, R¹ und R² die in Anspruch 1 oder 2 angegebene Bedeutung haben,
gegebenenfalls in Gegenwart eines Verdünnungsmittels auf Temperaturen zwischen 40°C und 120°C.

## Claims

1. Process for preparing ar(alk)yluracils of the general formula (I) 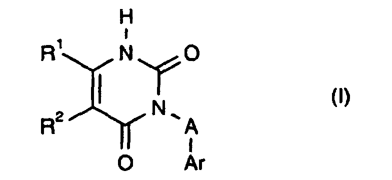 in which
A represents a single bond or represents straight-chain or branched alkanediyl having 1 to 4 carbon atoms,
Ar represents phenyl or naphthyl, each of which is optionally substituted by amino, cyano, halogen or the grouping -N(R³)SO₂R⁴,
R¹ represents optionally fluorine- and/or chlorine-substituted alkyl having 1 to 4 carbon atoms,
R² represents hydrogen, fluorine, chlorine, bromine or alkyl having 1 to 4 carbon atoms,
R³ represents hydrogen or represents in each case optionally fluorine- and/or chlorine-substituted alkyl, alkylcarbonyl or alkylsulphonyl having in each case 1 to 6 carbon atoms in the alkyl groups, represents in each case optionally fluorine- and/or chlorine-substituted cycloalkylcarbonyl or cycloalkylsulphonyl having in each case 3 to 6 carbon atoms in the cycloalkyl groups, or represents in each case optionally fluorine- and/or chlorine-substituted phenylcarbonyl or phenylsulphonyl, and
R⁴ represents optionally fluorine- and/or chlorine-substituted alkyl having 1 to 6 carbon atoms, represents optionally fluorine- and/or chlorine-substituted cycloalkyl having 3 to 6 carbon atoms, or represents in each case optionally fluorine- and/or chlorine-substituted phenyl,
**characterized in that** substituted aminoalkenamides of the general formulae (IIa) or (IIb) 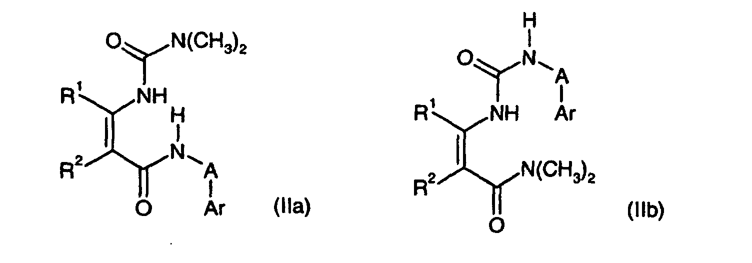 in which
A, Ar, R¹ and R² are as defined above
are heated at temperatures between 40°C and 120°C, if appropriate in the presence of a diluent.

2. Process according to Claim 1, **characterized in that**
A represents a single bond or represents a methylene group,
Ar represents phenyl which is optionally substituted by amino, cyano, fluorine, chlorine, bromine or the grouping -N(R³)SO₂R⁴,
R¹ represents in each case optionally fluorine- and/or chlorine-substituted methyl or ethyl,
R² represents hydrogen, fluorine, chlorine, bromine, methyl or ethyl,
R³ represents hydrogen or represents in each case optionally fluorine- and/or chlorine-substituted methyl, ethyl, n- or i-propyl, acetyl, propionyl, methylsulphonyl or ethylsulphonyl, represents in each case optionally fluorine- and/or chlorine-substituted cyclopropylcarbonyl, cyclobutylcarbonyl, cyclopentylcarbonyl, cyclohexylcarbonyl, cyclopropylsulphonyl, cyclobutylsulphonyl, cyclopentylsulphonyl or cyclohexylsulphonyl, or represents in each case optionally fluorine- and/or chlorine-substituted phenylcarbonyl or phenylsulphonyl, and
R⁴ represents in each case optionally fluorine- and/or chlorine-substituted methyl, ethyl, n- or i-propyl, n-, i-, s- or t-butyl, represents in each case optionally fluorine- and/or chlorine-substituted cyclopropyl, cyclobutyl, cyclopentyl or cyclohexyl, or represents in each case optionally fluorine- and/or chlorine-substituted phenyl.

3. Substituted aminoalkenamides of the general formulae (IIa) or (IIb) 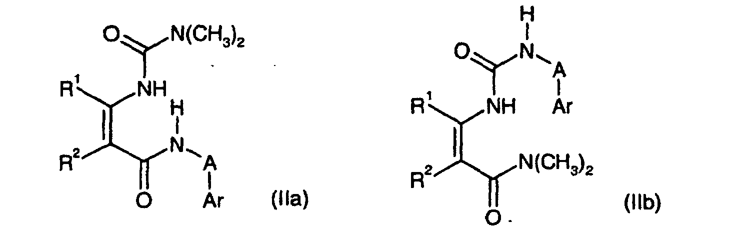 in which
A, Ar, R¹ and R² are as defined in Claim 1 or 2.

4. Process for preparing substituted aminoalkenamides of the general formula (IIa), 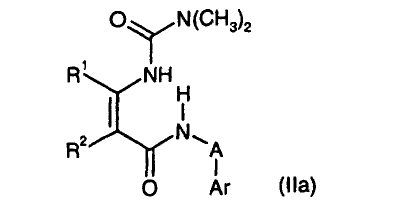 **characterized in that** substituted pyrimidones of the general formula (III) 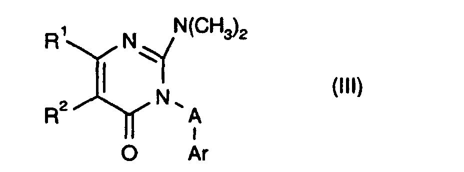 in which
A, Ar, R¹ and R² are as defined in Claim 1 or 2
are reacted at temperatures between 0°C and 100°C with an acid, if appropriate in the presence of a diluent.

5. Process for preparing substituted aminoalkenamides of the general formula (IIb) 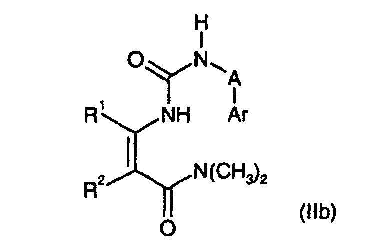 **characterized in that** substituted oxazinones of the general formula (IV) 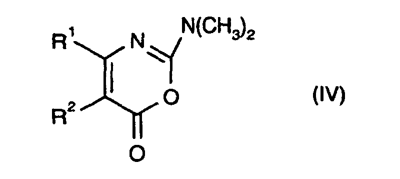 in which
R¹ and R² are as defined in Claim 1 or 2
are reacted with ar(alk)ylamines of the general formula (V)
H₂N-A-Ar (V)
in which
A and Ar are as defined in Claim 1 or 2
at temperatures between 0°C and 150°C, if appropriate in the presence of a diluent.

6. Substituted pyrimidones of the general formula (III) 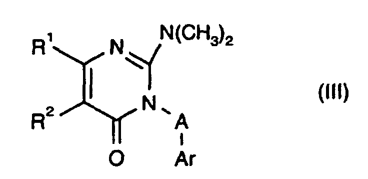 in which
A, Ar, R¹ and R² are as defined in Claim 1 or 2.

7. Process for preparing substituted pyrimidones of the general formula (III) 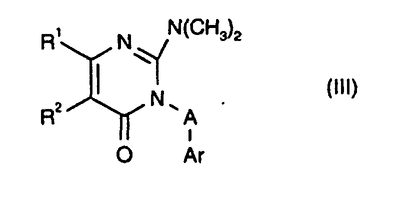 **characterized in that** substituted aminoalkenamides of the general formula (VI) 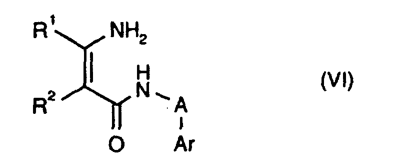 in which
A, Ar, R¹ and R² are as defined in Claim 1 or 2
are reacted with dichloromethylene-dimethylimmonium chloride of the formula (VII)
Cl₂C=N(CH₃)₂ Cl (VII)
at temperatures between 0°C and 100°C in the presence of a diluent.

8. Process for preparing ar(alk)yluracils of the general formula (I) 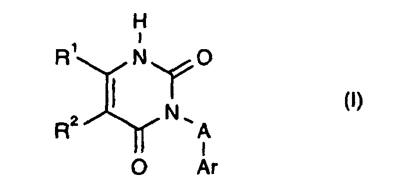 in which
A, Ar, R¹ and R² are as defined in Claim 1 or 2,
**characterized by** the following steps:
A) reaction of substituted aminoalkenamides of the general formula (VI) 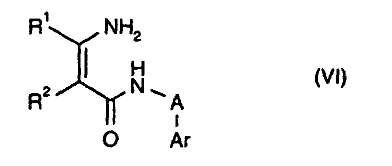 in which
A, Ar, R¹ and R² are as defined in Claim 1 or 2
with dichloromethylene-dimethylimmonium chloride of the formula (VII)
Cl₂C=N(CH₃)₂ Cl (VII)
at temperatures between 0°C and 100°C in the presence of a diluent;
B) reaction of the substituted pyrimidones of the general formula (III) prepared in step A) 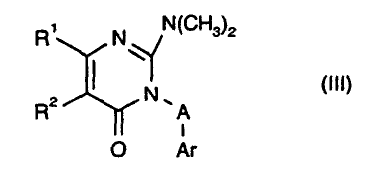 in which
A, Ar, R¹ and R² are as defined in Claim 1 or 2
at temperatures between 0°C and 100°C with an acid, if appropriate in the presence of a diluent, and
C) heating of the substituted aminoalkenamides of the general formulae (IIa) prepared in step B) 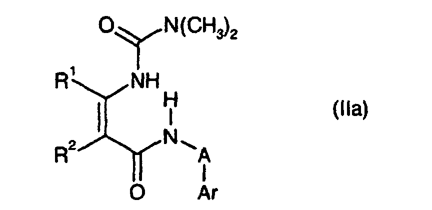 in which
A, Ar, R¹ and R² are as defined in Claim 1 or 2
at temperatures between 40°C and 120°C, if appropriate in the presence of a diluent.

## Revendications

1. Procédé de préparation d'ar(alco)yluraciles de la formule générale (I) : 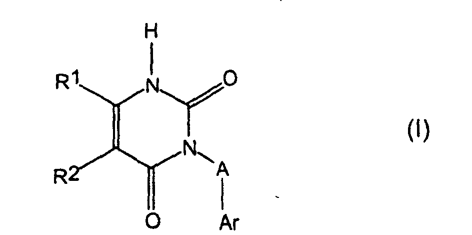 dans laquelle :
A représente une liaison simple ou un radical alcanediyle linéaire ou ramifié avec 1 à 4 atomes de carbone ;
Ar représente un radical phényle ou naphtyle chaque fois le cas échéant substitué par le radical amino, cyano, un atome d'halogène ou le groupement -N(R³)SO₂R⁴ ;
R¹ représente un radical alcoyle ayant 1 à 4 atomes de carbone, le cas échéant substitué par l'atome de fluor et/ou de chlore ;
R² représente l'atome d'hydrogène, de fluor, de chlore, de brome ou un radical alcoyle ayant 1 à 4 atomes de carbone ;
R³ représente l'atome d'hydrogène, un radical alcoyle, alcoylcarbonyle ou alcoylsulfonyle ayant 1 à 6 atomes de carbone dans le radical alcoyle, chaque fois le cas échéant substitué par l'atome de fluor et/ou de chlore, représente un radical cycloalcoylcarbonyle ou cycloalcoylsulfonyle ayant chaque fois 3 à 6 atomes de carbone dans le radical cycloalcoyle, chaque fois le cas échéant substitué par l'atome de fluor et/ou de chlore, ou représente un radical phénylcarbonyle ou phénylsulfonyle chaque fois le cas échéant substitué par l'atome de fluor et/ou de chlore, et
R⁴ représente un radical alcoyle, ayant 1 à 6 atomes de carbone, le cas échéant substitué par l'atome de fluor et/ou de chlore, représente un radical cycloalcoyle ayant 3 à 6 atomes de carbone, le cas échéant substitué par l'atome de fluor et/ou de chlore, ou représente un radical phényle le cas échéant substitué par l'atome de fluor et/ou de chlore,
**caractérisé en ce que** l'on fait chauffer l'amide substitué d'acide aminoalcénoïque de la formule générale (IIa) ou (IIb) : 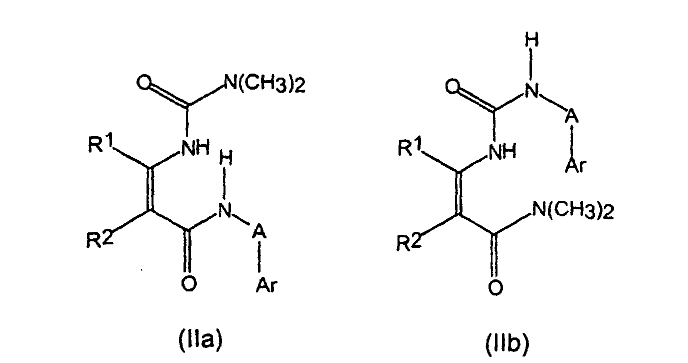 dans lesquelles :
A, Ar, R¹ et R² ont la signification indiquée ci-dessus,
le cas échéant en présence d'un agent de dilution, à des températures allant de 40°C à 120°C.

2. Procédé suivant la revendication 1, **caractérisé en ce que** :
A représente une simple liaison ou le radical méthylène ;
Ar représente un radical phényle le cas échéant substitué par le radical amino, cyano, l'atome de fluor, de chlore, de brome ou le groupement -N(R³)SO₂R⁴ ;
R¹ représente le radical méthyle ou éthyle chaque fois le cas échéant substitué par l'atome de fluor et/ou de chlore ;
R² représente l'atome d'hydrogène, de fluor, de chlore, de brome, le radical méthyle, éthyle ;
R³ représente l'atome d'hydrogène, le radical méthyle, éthyle, n- ou i-propyle, acétyle, propionyle, méthylsulfonyle ou éthylsulfonyle chaque fois le cas échéant substitué par l'atome de fluor et/ou de chlore, représente le radical cyclopropylcarbonyle, cyclobutylcarbonyle, cyclopentylcarbonyle, cyclohexylcarbonyle, cyclopropylsulfonyle, cyclobutylsulfonyle, cyclopentylsulfonyle ou cyclohexylsulfonyle chaque fois le cas échéant substitué par l'atome de fluor et/ou de chlore, ou représente le radical phénylcarbonyle ou phénylsulfonyle chaque fois le cas échéant substitué par l'atome de fluor et/ou de chlore, et
R⁴ représente le radical méthyle, éthyle, n- ou i-propyle, n-, i-, s- ou t-butyle chaque fois le cas échéant substitué par l'atome de fluor et/ou de chlore, représente le radical cyclopropyle, cyclobutyle, cyclopentyle ou cyclohexyle, chaque fois le cas échéant substitué par l'atome de fluor et/ou de chlore, ou représente le radical phényle le cas échéant substitué par l'atome de fluor et/ou de chlore.

3. Amides substitués d'acide aminoalcénoïque des formules générales (IIa) ou (IIb) : 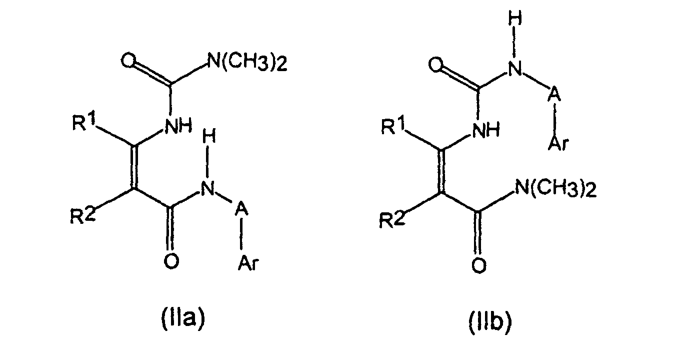 dans lesquelles :
A, Ar, R¹ et R² ont la signification indiquée à la revendication 1 ou 2.

4. Procédé de préparation d'amides substitués d'acide aminoalcénoïque de la formule générale (IIa) : 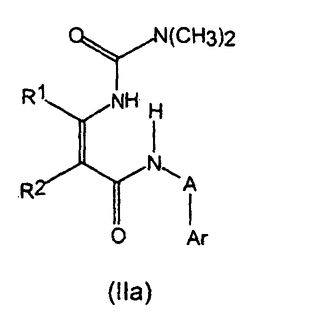 **caractérisé en ce que** l'on fait réagir les pyrimidones subtituées de la formule générale (III) : 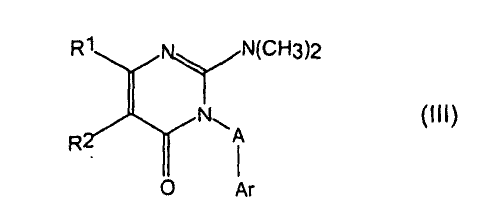 dans laquelle :
A, Ar, R¹ et R² ont la signification indiquée à la revendication 1 ou 2,
avec un acide, le cas échéant en présence d'un agent de dilution à des températures allant de 0°C à 100°C.

5. Procédé de préparation d'amides substitués d'acide aminoalcénoïque de la formule générale (IIb) : 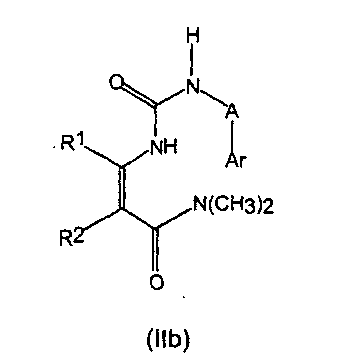 **caractérisé en ce que** l'on fait réagir les oxazinones subtituées de la formule générale (IV) : 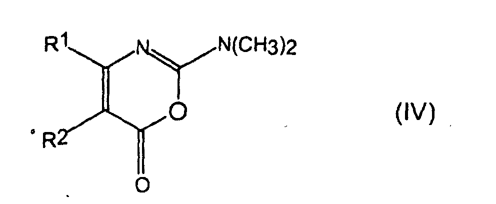 dans laquelle :
R¹ et R² ont la signification indiquée à la revendication 1 ou 2,
avec des ar(alco)ylamines de la formule générale (V) :
H₂N - A - Ar (V)
dans laquelle:
A et Ar ont la signification indiquée à la revendication 1 ou 2,
le cas échéant en présence d'un agent de dilution à des températures allant de 0°C à 150°C.

6. Pyrimidones substituées de la formule générale (III) : 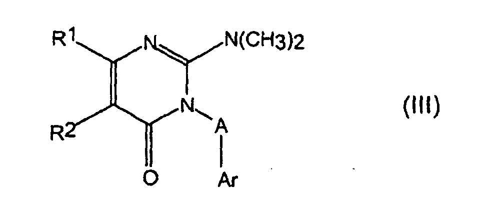 dans laquelle :
A, Ar, R¹ et R² ont la signification indiquée à la revendication 1 ou 2.

7. Procédé de préparation de pyrimidones substituées de la formule générale (III) : 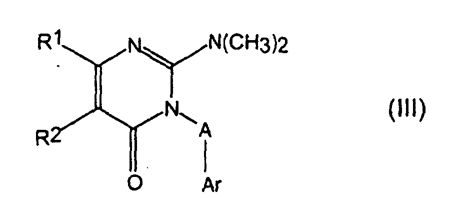 **caractérisé en ce que** l'on fait réagir des amides substitués d'acide aminoalcénoïque de la formule générale (VI) : 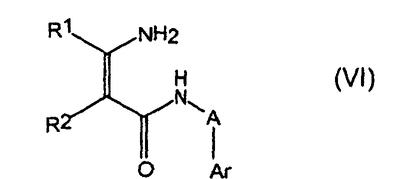 dans laquelle :
A, Ar, R¹ et R² ont la signification indiquée à la revendication 1 ou 2,
avec le chlorure de dichlorométhylènediméthylimmonium de la formule (VII) :
Cl₂C=N(CH₃)₂Cl (VII)
en présence d'un agent de dilution à des températures allant de 0°C à 100°C.

8. Procédé de préparation d'ar(alco)yluraciles de la formule générale (I) : 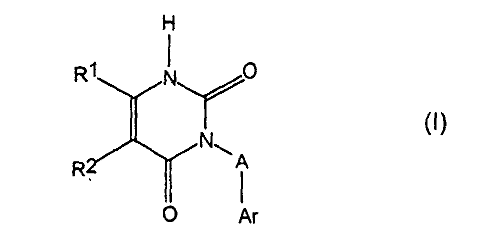 dans laquelle :
A, Ar, R¹ et R² ont la signification indiquée à la revendication 1 ou 2, **caractérisé par** les étapes suivantes de :
A) réaction des amides substitués d'acide aminoalcénoïque de la formule générale (VI) : 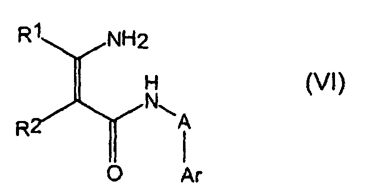 dans laquelle :
A, Ar, R¹ et R² ont la signification indiquée à la revendication 1 ou 2,
avec le chlorure de dichlorométhylènediméthylimmonium de la formule (VII) :
Cl₂C=N(CH₃)₂Cl (VII)
en présence d'un agent de dilution à des températures allant de 0°C à 100°C ;
B) réaction de la pyrimidone substituée préparée à l'étape A) de la formule générale (III) : 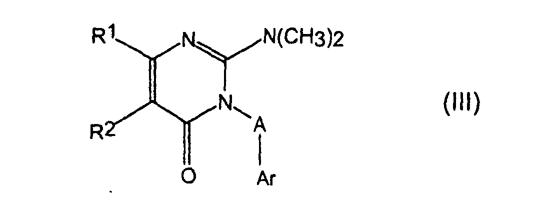 dans laquelle :
A, Ar, R¹ et R² ont la signification indiquée à la revendication 1 ou 2,
avec un acide, le cas échéant en présence d'un agent de dilution à des températures allant de 0°C à 100°C, et
C) chauffage de l'amide substitué d'acide aminoalcénoïque préparé à l'étape B), de la formule générale (IIa) : 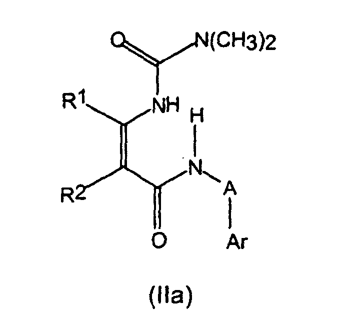 dans laquelle :
A, Ar, R¹ et R² ont la signification indiquée à la revendication 1 ou 2,
le cas échéant en présence d'un agent de dilution à des températures allant de 40°C à 120°C.
